Europäisches Patentamt

European Patent Office

Office européen des brevets

(11) Veröffentlichungsnummer: **0 575 951 A2**

# EUROPÄISCHE PATENTANMELDUNG

(21) Anmeldenummer: **93109910.5**

(22) Anmeldetag: **22.06.93**

(51) Int. Cl.5: **C12N 1/20**, //A23B4/20, A23L1/314, A23L1/318

(30) Priorität: **25.06.92 DE 4220889**

(43) Veröffentlichungstag der Anmeldung: **29.12.93 Patentblatt 93/52**

(84) Benannte Vertragsstaaten:
**AT BE CH DE DK ES FR GB GR IE IT LI LU MC NL PT SE**

(71) Anmelder: **Karl Müller & Co. KG**
**Klagenfurter Strasse 1-3**
**D-70469 Stuttgart(DE)**

(72) Erfinder: **Metz, Michael, Dr.**
**Alte Weinsteige 18**
**D-7000 Stuttgart 1(DE)**
Erfinder: **Meyer, Ingrid**
**Schellingstrasse 1**
**D-7253 Renningen 2(DE)**

(74) Vertreter: **Thiel, Christian, Dr. et al**
**Patentanwälte,**
**Herrmann-Trentepohl, Thiel, Kirschner,**
**Grosse, Bockhorni,**
**Schaeferstrasse 18**
**D-44623 Herne (DE)**

(54) **Reaktivierungsmischung für Starterkulturen zur Rohwurstherstellung.**

(57) Eine Reaktivierungsmischung für säurebildende und nitratreduzierende Starterkulturen zur Rohwurstherstellung insbesondere für Milchsäurebakterien, mit einer C-Quelle, einer N-Quelle, sowie bei der Kultivierung von Mikroorganismen üblichen Zusatzstoffen und Nährsalzen, enthält einen Puffer sowie die N-Quelle gegenüber der C-Quelle im Überschuß.

EP 0 575 951 A2

EP 0 575 951 A2

Die Erfindung betrifft eine Reaktivierungsmischung für säurebildende und nitratreduzierende Starterkulturen mit einer C-Quelle, einer N-Quelle, sowie für die Kultivierung von Mikroorganismen üblichen Zusatzstoffen und Nährsalzen Die Reaktivierungsmischung ist insbesondere zum Starten von Milchsäurebakterien für die Rohwurstherstellung bzw. -reifung geeignet.

Bei der Herstellung von schnittfesten und streichfähigen Rohwurstsorten, etwa Salami, werden seit vielen Jahren Starterkulturen für deren Herstellung verwendet. Starterkulturen sind in der Regel aus entsprechenden Lebensmitteln isolierte Mikroorganismen, die eine Rolle bei der Herstellung des Lebensmittels spielen und/oder diesen besonders erwünschte Eigenschaften verleihen. Beispiele sind die Säure- und Aromabildung bei der Herstellung von Joghurt, Salami und Sauerkraut, wie auch die Umrötung bei der Salamiherstellung.

Die Herstellung von Starterkulturen erfolgt in der Regel in großtechnischem Maßstab in biotechnologischen Anlagen, sogenannten Fermentern. Zur Konservierung werden die Mikroorganismen eingefroren, gefriergetrocknet oder thermisch getrocknet. Starterkulturen, die für den Einsatz bei der Rohwurstreifung bestimmt sind, werden üblicherweise gefriergetrocknet. Gefriergetrocknete Bakterien lassen sich sehr einfach bei Zimmertemperatur oder besser noch bei tiefen Temperaturen lagern und weisen ein einfaches Handling bei der Wurstherstellung auf.

Ein großer Nachteil von gefriergetrockneten oder thermisch getrockneten Starterkulturen bei der Wurstherstellung ist die Lag-Phase nach der Rehydratisierung. Trocknungsbedingt beginnt das erneute Wachstum des Mikroorganismus nicht unmittelbar nach der Rehydratisierung, sondern erst nach einiger Zeit. Diese Lag-Phase dauert eine bis mehrere Stunden und führt dazu, daß bei der Reifung von Rohwurst die zugesetzten Mikroorganismen gegenüber der sogenannten Spontanflora, also der Mikroorganismenflora, die sich bereits auf dem Fleisch befindet, einem Wachstumsnachteil unterliegt. Die eigentliche Aufgabe der Starterkultur, nämlich das Verdrängen der Spontanflora, kann daher nicht in idealer Weise geschehen.

Zudem werden die Mikroorganismen beim Einbringen in das jeweilige Substrat hohen Streßbedingungen ausgesetzt. So werden beispielsweise die gerade im Aufbau des Stoffwechsels befindlichen Organismen bei der Einbringung in ein Rohwurstgemenge in eine Umgebung mit Temperaturen um den Gefrierpunkt gebracht, welche, je nach angewandtem Verfahren, u.U. lokal hohe Konzentrationen an Salz und Gewürzen enthält. Diese Streßbedingungen wirken sich insbesondere dann aus, wenn der Mikroorganismus bei der Zugabe noch nicht voll aktiviert ist.

Der Erfindung liegt daher die Aufgabe zugrunde, die vorstehend beschriebene Lag-Phase von Starterkulturen nach der Rehydratisierung zu vermeiden, so daß sie im jeweils interessierenden Substrat von Anfang an voll aktiv vorliegen.

Diese Aufgabe wird mit einer Reaktivierungsmischung der eingangs genannten Art gelöst, die einen Puffer enthält und bei der die N-Quelle gegenüber der C-Quelle im Überschuß vorhanden ist.

Die Erfindung betrifft ferner ein Verfahren zur Reaktivierung von säurebildenden und nitratreduzierenden Starterkulturen, bei dem man den Mikroorganismus in Trockenform in eine erfindungsgemäße Reaktivierungsmischung gibt und dort über einen definierten Zeitraum reaktiviert.

In der erfindungsgmeäßen Reaktivierungsmischung wird der Mikroorganismus vor Zugabe zum zu fermentierenden Material, beispielsweise Fleischbrät oder Rohwurstgemenge, rehydratisiert und so lange gehalten, bis er eine, im Vergleich zum Status der herkömmlich reaktivierten Starterkultur, hohe Stoffwechselaktivität erreicht. Das Kriterium ist dabei der Säuerungsstoffwechsel. Herkömmlich rehydratisierte Starterkulturen werden vor der Zugabe in das Rohwurstgemenge lediglich in wenig Wasser aufgelöst und erfahren dabei keine nennenswerte Aktivierung.

Der über die erfindungsgemäße Reaktivierungsmischung reaktivierte Mikroorganismus besitzt bei Zugabe zu dem Substrat bereits ein sehr hohes Stoffwechselniveau und ist damit in der Lage, sich direkt nach Einbringen zu vermehren. Somit besitzt der Starterorganismus zum Zeitpunkt des Animpfens des Substrats gleiche Vorausetzungen, wie die Spontanflora. Eine optimale Funktion der Starterkultur ist hierdurch gewährleistet.

Die Reaktivierungsmischung erhält zweckmäßigerweise Glukose als Kohlenstoffquelle. Als Stickstoffquelle kommen insbesondere Protein- und Hefeextrakte in Frage, zweckmäßigerweise eine Mischung von beiden. Eine geeignete Stickstoffquelle ist beispielsweise Collagen.

Durch die Verwendung eines hohen Überschusses der N-Quelle im Medium gegenüber der C-Quelle, wobei das Verhältnis zweckmäßigerweise höchstens 1:2 beträgt und vorzugsweise im Bereich von 1:3 bis 1:7 liegt, jeweils auf das Gewicht bezogen, werden dem Mikroorganismus optimale Bedingungen geboten. Es handelt sich dabei um eine unübliche Zusammensetzung des Nährmediums, da üblicherweise die C-Quelle in nahezu gleichem Verhältnis oder im Überschuß, bezogen auf N-Quelle, zugegeben wird. Die spezielle Zusammensetzung des Reaktivierungsmediums ergibt bei vollständiger Zehrung der Kohlenstoffquelle einen starken Überschuß der N-Quelle, was für die Rekonstituierung des Stoffwechsels des jeweili-

2

gen Mikroorganismus optimal ist.

Neben der C-Quelle und der N-Quelle enthält die erfindungsgemäße Reaktivierungsmischung übliche Zusatzstoffe und Nährsalze, beispielsweise $MgSO_4$ und $MnSO_4$. Außerdem ist ein Puffer zugegen, beispielsweise Natriumhydrogencarbonat ($NaHCO_3$) oder auch ein Phosphat ($K_2HPO_4$ bzw. $Na_2HPO_4$). Der Puffer ist zweckmäßigerweise mengenmäßig auf die C-Quelle abgestimmt.

Die erfindungsgemäße Reaktivierungsmischung ist insbesondere für die Reaktivierung von getrockneten Milchsäurebakterien geeignet, welche Glukose nahezu quantitativ in Lactat (Milchsäure) umwandeln. Durch die vorgegebene unüblich geringe Glukosemenge und über den im Medium enthaltenen Puffer, insbesondere Phosphat und Hydrogencarbonat, wird ein minimaler pH-Wert von 4,7 bis 5,5 erreicht, bei welchem die Milchsäurebakterien auch bei längerem Verbleib in der Lösung keiner pH-Stase unterliegen. Die stationäre Phase des Mikroorganismenwachstums wird somit durch die gewollt herbeigeführte Limitierung der C-Quelle erreicht und nicht durch eine pH-Stase.

Durch in der Medienrezeptur enthaltene puffernde Substanzen, etwa Dikaliumhydrogenphosphat und Natriumhydrogencarbonat, wird eine Stabilisierung des pH-Wertes in der Lösung erreicht. Dadurch kann ein höherer C-Quellenanteil eingesetzt werden, als es ohne Pufferung möglich wäre. Eine größere Menge einer C-Quelle ohne Pufferung würde zu stark absinkenden pH-Werten von etwa 4,0 führen, was die pH-Stase der Kultur herbeiführen würde. Puffermenge und C-Quellenmenge bedingen daher einander.

Der Puffer wird zweckmäßigerweise in einer Menge von 0,5 bis 20 Gew.-%, bezogen auf das Trockengewicht der Reaktivierungsmischung, insbesondere 1,5 bis 15 Gew.-%, zugesetzt. Besonders bevorzugt als Puffer ist Natriumhydrogencarbonat.

Der C-Quellenanteil in der erfindungsgemäßen Reaktivierungsmischung beträgt vorzugsweise 5 bis 40 Gew.-%, insbesondere 8 bis 25 Gew.-%, und der N-Quellenanteil vorzugsweise 25 bis 85 Gew.-%, insbesondere 50 bis 75 Gew.-%, jeweils bezogen auf das Trockengewicht der Mischung. Die Mischung kann ferner bis zu 25 Gew.-%, bezogen auf das Trockengewicht, weitere übliche Zusätze und Nährstoffe enthalten.

Nachfolgend wird die Erfindung im einzelnen anhand von Reaktivierungsmischungen für Starterkulturen für die Rohwurstherstellung beschrieben, wobei Glucose als C-Quelle verwandt wird und Protein/Hefeextrakt als N-Quelle.

Durch den Einsatz der erfindungsgemäßen Reaktivierungsmischung wird das Keimwachstum in der Anfangsphase der Rohwurstreifung stark beschleunigt. So lag in Parallelversuchen mit und ohne Reaktivierungsmischung die Keimzahl bei reaktivierten Ansätzen um einen Faktor von bis zu 50 höher, als bei nicht reaktivierten Ansätzen. Dies führt zu einer besseren mikrobiologischen Absicherung der Rohwurst durch die definierte Starterkultur, der durch den Reaktivierungsprozeß eine stark verbesserte Konkurrenzfähigkeit gegenüber der Spontanflora verliehen wird.

Parallel zum beschleunigten Keimwachstum der reaktivierten Starterkultur unmittelbar nach der Zugabe zum Substrat ergibt sich eine Erhöhung der Säuerungsaktivität um einen Faktor von bis zu 2. Dies bedeutet eine schnellere Absäuerung der Rohwurst direkt vom Reifungsbeginn an.

Zweckmäßigerweise zeigt ein in der Reaktivierungsmischung befindlicher Farbindikator die Einsatzfähigkeit der reaktivierten Starterkultur an. Wie oben beschrieben, wird durch die Säurebildung der Bakterien der pH-Wert abgesenkt. Dabei muß ein Absinken auf solche Werte vermieden werden, die zu einer pH-Stase des Mikroorganismus führen. Dies ist bei Milchsäurebakterien spätestens bei einem pH-Wert von 4,0 der Fall. Der Farbindikator wird daher zweckmäßigerweise so gewählt, daß der Farbumschlag bei pH-Werten erfolgt, die deutlich darüber liegen. Hier haben sich pH-Werte von 5,7 bis 4,8 als zweckmäßig erwiesen.

Da sich der Farbindikator insbesondere auch in der Reaktivierungsmischung selbst befindet und diese Reaktivierungsmischung mit dem aktivierten Mikroorganismus in die Rohwurstmasse gegeben wird, ist eine lebensmittelrechtliche Unbedenklichkeit des Farbindikators erforderlich. Dieses Kriterium wird von Anthocyanfarbstoffen erfüllt, die zudem einen Farbumschlag bei den erwünschten pH-Werten von 5,7 bis 4,8 von Graublau nach Graurot zeigen. Der Umschlagspunkt des Farbindikators ist mit der Menge der Kohlenstoffquelle in der Reaktivierungsmischung so abgestimmt, daß bei Erreichen des Umschlags-pH-Wertes im wesentlichen die gesamte Kohlenstoffquelle aufgebraucht ist. Nach Verstoffwechselung der Kohlenstoffquelle besitzen die Mikroorganismen gute Aktivität für das Einbringen in die Rohwurstmasse.

Der Farbindikator erlaubt dem Anwender eine schnelle visuelle Kontrolle der Einsatzbereitschaft seiner reaktivierten Mikroorganismen. Bei noch nicht erfolgter Farbänderung kann der Anwender davon ausgehen, daß die Reaktivierung a) noch nicht beendet ist oder aber b) aufgrund inaktiver Starterkulturen nicht funktioniert. Insoweit stellt der Farbindikator gleichzeitig einen Funktionsindikator für die Starterkultur dar.

Die Reaktivierungsmischung liegt vorzugsweise in Form einer ggf. steril verpackten Trockenmischung vor. Dabei besteht die Verpackung zweckmäßigerweise aus einem Behälter mit definiertem Volumen, etwa 0,5 oder 1,1, beispielsweise in Form einer Flasche. Auf diesem Behälter angebrachte Markierungen erlauben das genaue Auffüllen auf das für die Reaktivierungsmischung jeweils geforderte Volumen.

Die als Trockenmischung vorliegende Reaktivierungsmischung kann die jeweilige Starterkultur in getrockneter Form zugesetzt oder separat verpackt enthalten.

Zweckmäßigerweise befindet sich auf dem Behälter, der die Reaktivierungsmischung als Trockenmischung enthält, eine Temperaturanzeige, etwa ein Maximumthermometer. Bei Verwendung von zu heißem Wasser zur Herstellung der Reaktivierungslösung, etwa von mehr als 40 ° C, wird dieses angezeigt und der Anwender auf die Möglichkeit einer Schädigung der darin befindlichen oder der Löschung zuzusetzenden Mikroorganismen aufmerksam gemacht. In der Regel sollte die Wassertemperatur 30 bis 35 ° C nicht überschreiten.

Zur visuellen Kontrolle der Einhaltung der Kulturbedingungen kann sich auf dem Behälter ein Feld mit einem Farbindikator befinden, der sich bei Überschreiten der maximal zulässigen Temperatur, etwa 40 ° C, dauerhaft verfärbt und somit die Unbrauchbarkeit des Ansatzes anzeigt.

Die erfindungsgemäß reaktivierte Starterkultur ist über einen Zeitraum von 8 Stunden aktiv und verwendbar. Sie kann beispielsweise zu Beginn des Arbeitstages angesetzt werden und über den gesamten Arbeitstag hinweg eingesetzt werden. Die Kultur verliert ihre Eigenschaften etwa 10 bis 15 Stunden nach erfolgter Reaktivierung, da sich der Mikroorganismus dann, bedingt durch die Substratlimitierung, zu lange in einer Lag-Phase befindet.

Mit der erfindungsgemäßen Reaktivierungsmischung wird der jeweilige Mikroorganismus vor seinem bestimmungsgemäßen Einsatz auf ein hohes Stoffwechselniveau gebracht. Von diesem hohen Stoffwechselniveau, das sich noch vor dem Teilungsstadium befindet, kann der Mikroorganismus dann unmittelbar nach Einbringen in die Rohwurstmasse, mit der Zellteilung beginnen, wodurch eine Gleichstellung der Starterkultur mit der Spontanflora besteht. Bedingt dadurch kann die reaktivierte Starterkultur die Spontanflora stärker zurückdrängen als eine nicht reaktivierte Starterkultur. Dies bringt eine gute Absicherung des Produkts mit sich.

Im allgemeinen beträgt die Reaktivierungszeit etwa 2 Stunden, abhängig von der gewählten Temperatur. Die Reaktivierung kann also an einem normalen Arbeitstag vor Arbeitsbeginn durchgeführt werden.

Bei Gegenwart eines pH-wertabhängigen, lebensmittelzugelassenen Farbindikators ist eine visuelle Beurteilung des Standes der Reaktivierung und der Funktion der Kultur möglich. Über den Farbumschlag kann die erfolgreiche Reaktivierung des Mikroorganismus ohne weitere Hilfsmittel sofort erkannt werden. Bei Fehlfunktion der Starterkultur findet in der Reaktivierungsmischung keine Farbänderung statt. Die Reaktivierungsmischung ist ein Nährmedium definierter Zusammensetzung, das beim Startermikroorganismus insbesondere den Stoffwechsel ermöglicht. Aufgrund der Limitierung der Kohlenstoffquelle und der Gegenwart des Puffers können die Organismen jedoch nur so viel Säure erzeugen, daß das Medium einen pH-Wert weit unter pH 5 nicht mehr erreichen kann. Auf diese Weise wird eine pH-Stase vermieden wie auch eine dadurch bedingte und unerwünschte Lag-Phase nach Einbringung des Mikroorganismus in das Produkt.

Das Überangebot an Protein in der Reaktivierungsmischung gewährleistet eine optimale Substratversorgung für die Rehydratisierungs- und Aktivierungsphase.

Die Menge der Reaktivierungsmischung und ihr Flüssigkeitsvolumen muß individuell auf die zu reaktivierende Menge der Starterkultur abgestimmt sein. Wichtig ist dabei die Abstimmung von Puffermenge und Kohlenstoffquelle zur Einhaltung eines unteren pH-Wertes von etwa 5.

Die Erfindung wird durch die nachfolgenden Beispiele bevorzugter Ausführungsformen näher erläutert.

Beispiel 1

Zwei Reaktivierungsmischungen I und II wurden gemäß nachstehender Tabelle durch Einrühren der Trockensubstanzen in Wasser und Auffüllen auf 500 ml hergestellt. Dabei ist die Aktivierungsmischung I zum Starten von Reinstammstarterkulturen (LB) bestimmt, die Mischung II für Starterkulturmischungen (LS).

In beiden Mischungen I und II wurden Starterkulturen bei 30 bis 35 ° C in verschlossenen Flaschen aktiviert. Nach zwei Stunden erfolgt der Farbumschlag des Anthocyanfarbstoffes von grau nach rot. Anschließend wurde die Starterkultur durch heftiges Schütteln von der Flaschenwandung gelöst. Der Inhalt einer jeden Flasche reicht für 100 kg Rohwurstmasse aus.

Es ist festzuhalten, daß durch Einsatz der erfindungsgemäßen Reaktivierungsmischung die Zugabemenge an Starterkultur zur Rohwurstmasse nicht vermindert werden kann, da während der Reaktivierung eine Keimvermehrung nicht oder nur in geringem Maße stattfindet.

| Reinstammstarterkulturen | | | | |
|---|---|---|---|---|
| | Unterer Grenzwert | | Oberer Grenzwert | |
| | g | % Anteil | g | % Anteil |
| Kollagenhydrolisat | 3 | 26,55 | 7 | 32,76 |
| Hefeextrakt | 3 | 26,55 | 7 | 32,76 |
| Glucose | 2 | 17,70 | 2 | 9,36 |
| NaHCO3 | 1 | 8,85 | 1 | 4,68 |
| Reinlecithin | 0,25 | 2,21 | 1 | 4,68 |
| Anthocyanfarbstoff | 0,5 | 4,42 | 0,8 | 3,74 |
| MgSO4 | 0,05 | 0,44 | 0,07 | 0,33 |
| MnSO4 | 1,5 | 13,27 | 2,5 | 11,70 |
| Gesamt | 11,3 | 100,00 | 21,37 | 100,00 |

| Starterkulturmischungen | | | | |
|---|---|---|---|---|
| | Unterer Grenzwert | | Oberer Grenzwert | |
| | g | % Anteil | g | % Anteil |
| Kollagenhydrolisat | 3 | 30,53 | 7 | 37,00 |
| Hefeextrakt | 3 | 30,53 | 7 | 37,00 |
| Glucose | 2 | 20,36 | 2 | 10,57 |
| NaHCO3 | 1 | 10,18 | 1 | 5,29 |
| Reinlecithin | 0,25 | 2,54 | 1 | 5,29 |
| Anthocyanfarbstoff | 0.5 | 5,09 | 0,8 | 4,23 |
| MgSO4 | 0,05 | 0,51 | 0,07 | 0,37 |
| MnSO4 | 0,025 | 0,25 | 0,05 | 0,26 |
| Gesamt | 9,825 | 100,00 | 18,92 | 100,00 |

Beispiel 2

Rohwurstmasse wurde nach folgender Rezeptur hergestellt:

| Berechnung pro 100 kg | | |
|---|---|---|
| **Rohmaterial:** | 35 kg | R3 gewolft und gekühlt |
| | 35 kg | S3 gewolft und gefroren |
| | 30 kg | S8 kleingeschnitten und gefroren |

| Zugabe pro 100 kg | | |
|---|---|---|
| **Gewürze:** | 28 g | NaCl + NPS (LB-Mischungen) |
| | 28 g | NaCl (LS-Mischungen) |
| | 0,3 g | Salpeter |
| | 2 g | Pfeffer |
| | 5 g | Rosalin. |

Die Rohwurstmasse wurde zu je 5 kg portioniert und parallel zueinander mit Starterkulturen gemäß Beispiel 1 versetzt, wobei erfindungsgemäß reaktivierte und nicht reaktivierte Kulturen einander gegenübergestellt wurden. Jedem der Ansätze wurden 25 ml Starterkulturlösung zugesetzt. Dabei wurden die Starterkulturen der Kontrolle ohne Reaktivierung direkt vor dem Einsatz in das Rohwurstgemisch in 500 ml Leitungswasser gelöst. Aus jeder Charge wurden 10 Würste a 500 g hergestellt.

Die Reifung aller Würste erfolgte in derselben Reifekammer, gemäß nachfolgenden Klimatisierungsbedingungen:

| Stunden | Temperatur | LF (%) |
|---------|------------|--------|
| 0 | 23° C | 70-80 % |
| 9 | 23° C | 90-92 % |
| 17 | 23° C | 90-92 % |
| 25 | 23° C | 88-90 % |
| 37 | 18° C | 90 % |
| 49 | 16-18° C | 86-88 % |
| 73 | 13-15° C | 84-86% |

In allen Ansätzen wurden Milchsäure erzeugende Starterkulturen verwandt, zum Vergleich jeweils mit und ohne Reaktivierung in einer erfindungsgemäßen Mischung. Es wurden acht Starterkulturen mit und ohne Reaktivierung eingesetzt, mit den folgenden Bezeichnungen:

1. LB 25 ohne Reaktivierung
2. LB 25 mit Reaktivierung
3. LS 25 ohne Reaktivierung
4. LS 25 mit Reaktivierung
5. LB 3 ohne Reaktivierung
6. LB 3 mit Reaktivierung
7. LS 3 ohne Reaktivierung
8. LS 3 mit Reaktivierung.

Gemessen wurden in den einzelnen Ansätzen zum angegebenen Zeitpunkt die pH-Werte (Handmessung mit pH-Meter und Stichelektrode, jeweils Doppelmessung) und die Keimzahl der angegebenen Mikroorganismen (gemäß Analysenvorschrift: Keimzahlbestimmung in Rohwurst, Fa. Gewürzmüller, Stuttgart, 1991).

Die ermittelten Daten sind in Tabelle 3 wiedergegeben, in der die Kürzel o.R und m.R für ohne bzw. mit Reaktivierung stehen, LAC und LAD Laktobazillenstämme und SAB einen Staphylokokkenstamm bezeichnen, die jeweils zu Starterzwecken zugegeben wurden.

## Tabelle 1

**Auswirkung der Keimreaktivierung auf Keimwachstum und Säuerung in standardisierter Rohwurst**

| Ansatz-bezeichnung | | Parameter | | 0 Tage | Fakt. | 0 Tage | Fakt. | 1 Tag | Fakt. | 1 Tag | Fakt. |
|---|---|---|---|---|---|---|---|---|---|---|---|
| | | | | | | | | Zeit in Stunden | | | |
| | | | | 0 | | 9 | | 17 | | 25 | |
| LB-25 | o.R | pH-Wert | | 6,25 | 1,00 | 6.28 | 1,00 | 6,18 | 1,00 | 5.74 | 1,00 |
| | | Laktobacillen | spont. | 2,3E+05 | 1,00 | 3,5E+05 | 1,00 | 2.5E+05 | 1,00 | 1,0E+05 | 1,00 |
| | | LAD | kult. | 1,9E+06 | 1,00 | 2,5E+06 | 1,00 | 1,1E+07 | 1,00 | 4,9E+08 | 1,00 |
| LB-25 | m.R | pH-Wert | | 6,25 | 1,00 | 6.26 | 1,00 | 5,92 | 0,96 | 5.35 | 0,93 |
| | | Laktobacillen | spont. | 1,8E+05 | 0,78 | 4,0E+05 | 1,14 | 7,0E+04 | 0,28 | 1.0E+05 | 1,00 |
| | | LAD | kult. | 5,3E+06 | 2,79 | 9,6E+08 | 384 | 6,3E+08 | 57,27 | 9,5E+08 | 1,94 |
| LS-25 | o.R | pH-Wert | | 6,32 | 1,00 | 6.31 | 1,00 | 6,24 | 1,00 | 5.58 | 1,00 |
| | | Laktobacillen | spont. | 2,5E+05 | 1,00 | 9.0E+05 | 1,00 | 7.2E+05 | 1,00 | 2.3E+06 | 1,00 |
| | | LAD | kult. | 1,7E+06 | 1,00 | 2,5E+06 | 1,00 | 5.2E+07 | 1,00 | 5.6E+08 | 1,00 |
| | | SAB | kult. | 3,4E+06 | 1,00 | 2,8E+06 | 1,00 | 7,9E+06 | 1,00 | 6.3E+06 | 1,00 |
| LS-25 | m.R | pH-Wert | | 6,25 | 0,99 | 6.28 | 1,00 | 5,98 | 0,96 | 5.35 | 0,96 |
| | | Laktobacillen | spont. | 3,0E+05 | 1,20 | 3,0E+05 | 0,33 | 6,5E+05 | 0,90 | 5.0E-04 | 0,02 |
| | | LAD | kult. | 4,3E+06 | 2,58 | 1,4E+07 | 5,60 | 3,4E+08 | 6,54 | 1.2E-09 | 2,14 |
| | | SAB | kult. | 3,6E+06 | 1,06 | 2,8E+06 | 1,00 | 1,2E+07 | 1,52 | 3,8E+06 | 0,60 |
| LB-3 | o.R | pH-Wert | | 6,27 | 1,00 | 6.28 | 1,00 | 6.15 | 1,00 | 5.72 | 1,00 |
| | | Laktobacillen | spont. | 3,0E+05 | 1,00 | 1,5E+05 | 1,00 | 8,5E+05 | 1,00 | 6.0E-05 | 1,00 |
| | | LAC | kult. | 2,6E+06 | 1,00 | 2,6E+06 | 1,00 | 1,4E+08 | 1,00 | 2,9E+08 | 1,00 |
| LB-3 | m.R | pH-Wert | | 6,27 | 1,00 | 6.25 | 1,00 | 6.08 | 0,99 | 5.5 | 0,96 |
| | | Laktobacillen | spont. | 4,0E+05 | 1,33 | 2,5E+05 | 1,67 | 3,0E+05 | 0,35 | 7,5E+05 | 1,25 |
| | | LAC | kult. | 2,8E+06 | 1,08 | 3,1E+06 | 1,19 | 1,2E+08 | 0,86 | 1,0E+09 | 3,45 |
| LS-3 | o.R | pH-Wert | | 6,25 | 1,00 | 6.26 | 1,00 | 6.2 | 1,00 | 5.46 | 1,00 |
| | | Laktobacillen | spont. | 1,3E+05 | 1,00 | 6,5E+05 | 1,00 | 2.2E+06 | 1,00 | 1.0E+06 | 1,00 |
| | | LAC | kult. | 2,2E+06 | 1,00 | 2,5E+06 | 1,00 | 6,4E+07 | 1,00 | 3,2E+08 | 1,00 |
| | | SAB | kult. | 9,0E+06 | 1,00 | 5,9E+06 | 1,00 | 1,8E+07 | 1,00 | 6,1E+06 | 1,00 |
| LS-3 | m.R | pH-Wert | | 6,27 | 1,00 | 6.28 | 1,00 | 6.09 | 0,98 | 5.36 | 0,98 |
| | | Laktobacillen | spont. | 1,1E+05 | 0,85 | 3,0E+05 | 0,46 | 7,5E+05 | 0,34 | 1,0E+06 | 1,00 |
| | | LAC | kult. | 2,9E+06 | 1,32 | 2,0E+06 | 0,80 | 2,2E+08 | 3,44 | 9,4E+08 | 2,94 |
| | | SAB | kult. | 6,1E+06 | 0,68 | 5,7E+06 | 0,97 | 2,7E+06 | 0,15 | 1,1E+07 | 1,80 |

## Tabelle 1 Fortsetzung

| Ansatz-bezeich-nung | | Parameter | | 1 Tag 30 | 1 Tag 37 | Fakt. | 2 Tage 49 | Fakt. | 3 Tage 73 | Fakt. |
|---|---|---|---|---|---|---|---|---|---|---|
| LB-25 | o.R | pH-Wert | | 5,71 | 5.32 | 1,00 | 5.17 | 1,00 | 5,15 | 1,00 |
| | | Laktobacillen | spont. | | 1,0E+07 | 1,00 | 7,5E+06 | 1,00 | 5,0E+06 | 1,00 |
| | | LAD | kult. | | 1,5E+09 | 1,00 | 1,1E+09 | 1,00 | 1,2E+09 | 1,00 |
| LB-25 | m.R | pH-Wert | | 5,23 | 5.14 | 0,97 | 5.17 | 1,00 | 5,17 | 1,00 |
| | | Laktobacillen | spont. | | 1,0E+06 | 0,10 | 1,5E+06 | 0,20 | 5,0E+05 | 0,10 |
| | | LAD | kult. | | 1,3E+09 | 0,90 | 9,2E+08 | 0,84 | 1,6E+09 | 1,33 |
| LS-25 | o.R | pH-Wert | | 5,32 | 5.21 | 1,00 | 5,1 | 1,00 | 5,12 | 1,00 |
| | | Laktobacillen | spont. | | 1,5E+06 | 1,00 | 7,5E+06 | 1,00 | 4,0E+06 | 1,00 |
| | | LAD | kult. | | 1,1E+09 | 1,00 | 9,6E+08 | 1,00 | 1,2E+09 | 1,00 |
| | | SAB | kult. | | 3,1E+06 | 1,00 | 2,7E+06 | 1,00 | 3,3E+06 | 1,00 |
| LS-25 | m.R | pH-Wert | | 5,2 | 5.08 | 0,98 | 5.13 | 1,01 | 5,14 | 1,00 |
| | | Laktobacillen | spont. | | 1,5E+06 | 1,00 | 1,5E+06 | 0,20 | 1,5E+06 | 0,38 |
| | | LAD | kult. | | 1,2E+09 | 1,09 | 1,1E+09 | 1,15 | 1,4E+09 | 1,17 |
| | | SAB | kult. | | 6,0E+06 | 1,94 | 6,6E+06 | 2,44 | 6,9E+06 | 2,09 |
| LB-3 | o.R | pH-Wert | | 5,37 | 5.2 | 1,00 | 5.12 | 1,00 | 5,15 | 1,00 |
| | | Laktobacillen | spont. | | 5,5E+06 | 1,00 | 4,5E+06 | 1,00 | 1,0E+07 | 1,00 |
| | | LAC | kult. | | 7,7E+08 | 1,00 | 9,0E+08 | 1,00 | 1,3E+09 | 1,00 |
| LB-3 | m.R | pH-Wert | | 5,23 | 5.06 | 0,97 | 5.06 | 0,99 | | 0,00 |
| | | Laktobacillen | spont. | | 1,5E+06 | 0,27 | 5,0E+05 | 0,11 | 4,0E+06 | 0,40 |
| | | LAC | kult. | | 1,3E+09 | 1,69 | 8,7E+08 | 0,97 | 1,1E+09 | 0,85 |
| LS-3 | o.R | pH-Wert | | 5,31 | 5.12 | 1,00 | 5.13 | 1,00 | 5,06 | 1,00 |
| | | Laktobacillen | spont. | | 1,5E+06 | 1,00 | 2,5E+06 | 1,00 | 1,5E+06 | 1,00 |
| | | LAC | kult. | | 1,0E+09 | 1,00 | 8,6E+08 | 1,00 | 1,0E+09 | 1,00 |
| | | SAB | kult. | | 1,0E+07 | 1,00 | 7,3E+06 | 1,00 | 8,8E+06 | 1,00 |
| LS-3 | m.R | pH-Wert | | 5,2 | 5.06 | 0,99 | 5.13 | 1,00 | 5,13 | 1,01 |
| | | Laktobacillen | spont. | | 1,0E+06 | 0,67 | 5,0E+05 | 0,20 | 2,5E+06 | 1,67 |
| | | LAC | kult. | | 9,7E+08 | 0,97 | 9,1E+08 | 1,06 | 1,2E+09 | 1,20 |
| | | SAB | kult. | | 1,7E+07 | 1,70 | 9,3E+06 | 1,27 | 1,1E+07 | 1,25 |

Durch den Einsatz einer reaktivierten Starterkultur liegen die Keimzahlen innerhalb der ersten 24h bis 48h der Rohwurstreifung deutlich über den Keimzahlen der nicht reaktivierten Starterkulturen. Hieraus ergibt sich eine sehr gute Absicherung des Lebensraumes Rohwurst, unmittelbar nach Beginn der Rohwurstreifung.

Dies bedeutet einen eindeutigen Vorteil hinsichtlich des Hygienestatus der Wurst speziell im Hinblick auf die Qualitätssicherung. Für die Aufgabe der Qualitätssicherung ist es von grundlegender Bedeutung, innerhalb kürzester Zeit eine definierte Flora in der Rohwurst zu etablieren. Die Keimzahl der spontanen Lactobazillen konnte durch Einsatz von reaktivierten Starterkulturen auf 10 bis 40 % der Keimzahl mit nicht reaktivierten Starterkulturen zurückgedrängt werden. Dies ist ein entscheidender Faktor im Hinblick auf die Qualitätssicherung über den Einsatz von Starterkulturen.

Die gleichlaufenden Parameter von reaktiviertem und nicht reaktiviertem Ansatz innerhalb der ersten 10 Stunden der Rohwurstreifung sind auf den Temperaturangleich der Wurst zurückzuführen. Nach 10h liegt die Rohwurst in einem für die Mikroorganismen günstigen Temperaturbereich über 15° C. Ab hier setzt der Reifungsprozeß ein und der bessere Status der reaktivierten Kulturen kann zur Geltung kommen.

8

Die bei den Ansätzen mit LB 25 und LS 25 ermittelten höheren Anfangskeimzahlen, bei den Ansätzen mit Reaktivierung, sind auf den besseren physiologischen Zustand der reaktivierten Starterkultur Zurückzuführen und nicht auf eine Vermehrung der Keime während der Reaktivierung. Eine Keimvermehrung während der Reaktivierung findet nicht statt.

Die Reduktion der Spontankeimzahl ist auf die hohe Stoffwechselaktivität der reaktiverten Starterkultur zurückzuführen, welche zu einer sehr schnellen Substratzehrung führt und der Spontanflora somit das Substrat entzieht.

Die Endkeimzahl der Starterkulturkeime in der Rohwurst ist mit und ohne reaktivierten Starterkulturen identisch. Aufgrund dessen wird die Gesamtcharakteristik sowie die Gesamtkeimzahl der Rohwurst durch Einsatz einer Reaktivierungsmischung nicht beeinflußt.

Die Keimzahl der Staphylokokken, welche als Starterkulturkeime in den Mischungen enthalten sind, liegt bei den reaktivierten Mischungen um den Faktor 1,3 bis 2 höher, im Vergleich zu nicht reaktivierten Mischungen. Dies zeigt einen ebenfalls positiven Einfluß der Reaktivierung auf die Staphylokokken, welche sich im Rahmen der Rohwurstreifung normalerweise nicht vermehren. Die reaktivierten Staphylokokken weisen einen besseren physiologischen Status auf. Aufgrund dessen ergibt sich auch bei den Staphylokokken eine höhere Wiederfindung.

Innerhalb der durchgeführten Ansätze wies die Mischung LS 3 mit Stamm LAC plus SAB die geringsten Unterschiede zwischen reaktiviertem und nicht reaktiviertem Ansatz auf, wobei eine positive Auswirkung der Reaktivierung bei dieser Starterkulturmischung deutlich erkennbar war. Bei allen anderen eingesetzten Starterkulturmischungen waren eindeutig positive Unterschiede bei den reaktivierten Starterkulturen erkennbar.

**Patentansprüche**

1. Reaktivierungsmischung für säurebildende und nitratreduzierende Starterkulturen, insbesondere für Milchsäurebakterien, mit einer C-Quelle, einer N-Quelle, sowie bei der Kultivierung von Mikroorganismen üblichen Zusatzstoffen und Nährsalzen, dadurch gekennzeichnet, daß sie einen Puffer enthält und die N-Quelle gegenüber der C-Quelle im Überschuß vorhanden ist.

2. Reaktivierungsmischung nach Anspruch 1, dadurch gekennzeichnet, daß sie Glukose als C-Quelle enthält.

3. Reaktivierungsmischung nach Anspruch 1 oder 2, dadurch gekennzeichnet, daß sie Protein und/oder Hefeextrakt als N-Quelle enthält.

4. Reaktivierungsmischung nach einem der vorstehenden Ansprüche, dadurch gekennzeichnet, daß das Gewichtsverhältnis C-Quelle : N-Quelle höchstens 1 : 2 ist.

5. Reaktivierungsmischung nach Anspruch 4, dadurch gekennzeichnet, daß das Gewichtsverhältnis im Bereich von 1 : 3 bis 1 : 7 liegt.

6. Reaktivierungsmischung nach einem der vorstehenden Ansprüche, dadurch gekennzeichnet, daß sie den Puffer in einer Menge von 0,5 bis 20 Gew.-%, vorzugsweise 1,5 bis 15 Gew.-%, bezogen auf das Trockengewicht der Reaktivierungsmischung, enthält.

7. Reaktivierungsmischung nach einem der vorstehenden Ansprüche, dadurch gekennzeichnet, daß sie $NaHCO_3$ und/oder $K_2HPO_4$ als Puffer enthält.

8. Reaktivierungsmischung nach einem der vorstehenden Ansprüche, gekennzeichnet durch einen C-Quellenanteil von 5 bis 40 Gew-%, vorzugsweise 8 bis 25 Gew.-% und einen N-Quellenanteil von 25 bis 85 Gew.-%, vorzugsweise 50 bis 75 Gew.-%, jeweils bezogen auf das Trockengewicht der Mischung.

9. Reaktivierungsmischung nach einem der vorstehenden Ansprüche, dadurch gekennzeichnet, daß sie einen pH-empfindlichen Farbstoff enthält.

10. Reaktivierungsmischung nach Anspruch 9, gekennzeichnet durch einen Anthoxyanfarbstoff mit einem Farbumschlag im Bereich von pH 4,8 bis 5,7.

11. Reaktivierungsmischung nach einem der vorstehenden Ansprüche in Form einer Trockenmischung

12. Reaktivierungsmischung nach Anspruch 11, dadurch gekennzeichnet, daß sie die Starterkultur in gefriergetrockneter Form zugesetzt enthält.

13. Reaktivierungsmischung nach Anspruch 11 oder 12, dadurch gekennzeichnet, daß sie in einem Behälter mit definiertem Volumen abgefüllt vorliegt.

14. Reaktivierungsmischung nach Anspruch 13, gekennzeichnet durch eine Temperaturanzeige auf dem Behälter.

15. Reaktivierungsmischung nach Anspruch 14, gekennzeichnet durch einen temperaturempfindlichen Farbindikator, der sich bei Überschreiten einer Temperatur von 35 bis 42° C verfärbt.

16. Verwendung einer Reaktivierungsmischung nach einem der vorstehenden Ansprüche für Lactobazillen als Starterkulturen, insbesondere für die Rohwurstherstellung.

17. Verfahren zur Reaktivierung von säurebildenden Starterkulturen, insbesondere von Milchsäurebakterien, dadurch gekennzeichnet, daß man den Mikroorganismus in Trockenform in eine Reaktivierungsmischung nach einem der Ansprüche 1 bis 15 gibt und darin über einen definierten Zeitraum wachsen läßt.